# FASCICULE DE BREVET EUROPEEN

(11) **EP 4 180 086 B1**
(45) Date de publication et mention de la délivrance du brevet: **18.06.2025**
(21) Numéro de dépôt: 22206561.7
(22) Date de dépôt: 10.11.2022
(51) Int. Cl.: A61M 60/178, A61M 60/859, A61M 60/863

(54) **DISPOSITIF DE FIXATION ET DE POSITIONNEMENT D'UNE POMPE CARDIAQUE**
VORRICHTUNG ZUR FIXIERUNG UND POSITIONIERUNG EINER HERZPUMPE
DEVICE FOR FIXING AND POSITIONING A HEART PUMP

(30) Priorité: 10.11.2021 FR 2111957
(43) Date de publication de la demande: 17.05.2023
(73) Titulaire: Fineheart, 33600 Pessac (FR)
(72) Inventeur: GARRIGUE, Stéphane, 33130 BEGLES (FR); ROUSSEAU, Jean-Baptiste, 33600 PESSAC (FR); BOUGERE, Emmanuel, 33600 PESSAC (FR); COLLAS, Jérémy, 33200 BORDEAUX (FR)
(74) Mandataire: Plasseraud IP

(56) Documents cités:
- WO-A1-2010/050114
- WO-A1-2018/052482
- FR-A1- 3 103 101
- US-A1- 2005 147 863
- US-A1- 2017 149 077
- US-A1- 2021 170 164

## Description

### Domaine technique

Le présent document concerne le domaine des pompes d'assistance cardiaque.

Elle concerne plus particulièrement un dispositif médical de fixation d'une pompe cardiaque dans une ouverture d'une paroi ventriculaire d'un cœur battant. Un tel dispositif médical de fixation d'une pompe cardiaque est décrit dans le document WO2010/050114A1.

### Technique antérieure

L'insuffisance cardiaque est une pathologie dans laquelle un cœur d'un patient présente une incapacité à fournir un débit sanguin suffisant pour répondre aux besoins métaboliques de l'organisme.

Des pompes d'assistance cardiaque sont classiquement utilisées pour assister un ventricule gauche d'un cœur. On parle alors de pompe cardiaque artificielle. Cette pompe artificielle et mécanique ne remplace pas le cœur qui continue de fonctionner mais apporte une aide au ventricule affaibli dans le but d'accroitre le débit sanguin de façon adaptée aux besoins de l'individu.

Dans le cas où une transplantation de greffon n'est pas possible, cette pompe cardiaque est implantée à long terme.

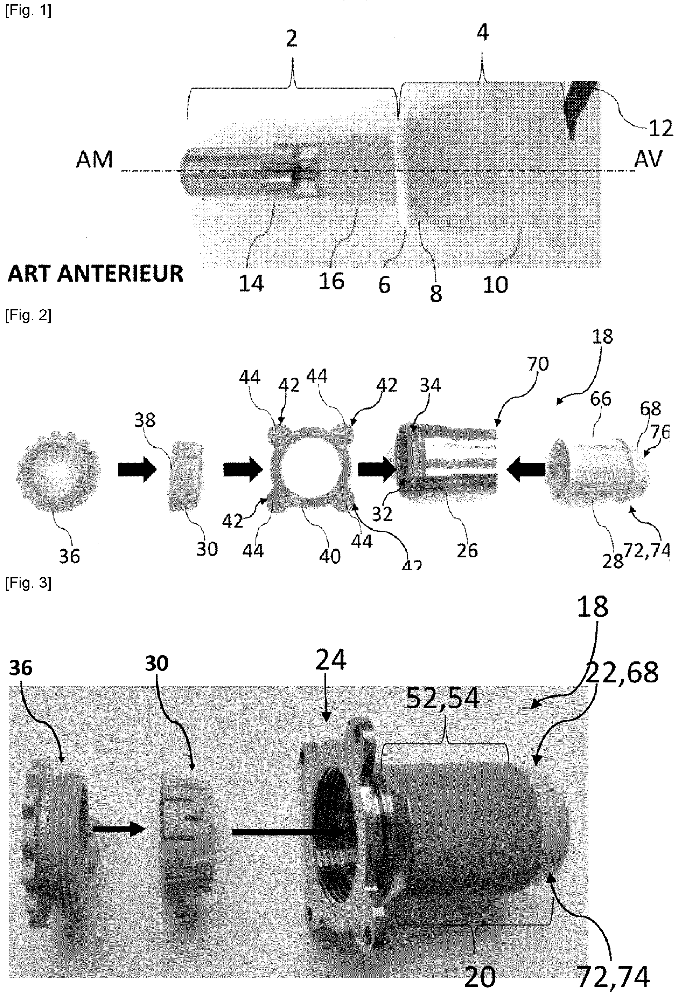

Comme illustré en figure 1, les pompes cardiaques implantables de l'état de l'art comportent typiquement une partie intraventriculaire 2 et une partie extraventriculaire 4. Dans la partie extraventriculaire 4, un dispositif médical de pompe cardiaque de l'état de l'art comprend d'amont (AM) en aval (AV) : une bride d'assemblage 6, un connecteur 8, un fourreau amovible avec clapet anti-retour 10 et un câble d'alimentation 12. Dans la partie intraventriculaire 2, ce dispositif médical comporte une pompe cardiaque 14 et un insert 16 réalisé en polyétheréthercétone (PEEK), ce dernier permettant de recevoir, supporter et orienter ladite pompe cardiaque 14 par rapport à la valve aortique. Les parties amont et aval sont ici repérées par rapport à la paroi ventriculaire du cœur battant.

Cependant avec un tel dispositif médical, on observe une colonisation cellulaire de l'insert 16 qui s'étend à la partie en saillie du corps de la pompe cardiaque 14. Une obstruction des orifices d'aspiration du sang de la pompe cardiaque 14 apparait ainsi dans le temps entraînant de moindres performances de la pompe cardiaque, lesquelles peuvent avoir de graves conséquences pour la santé du patient.

Il existe donc un besoin pressant pour un insert permettant de surmonter les inconvénients de l'art antérieur.

### Objet de l'invention

La présente invention vise à pallier les inconvénients de l'art antérieur en proposant un dispositif de fixation d'une pompe cardiaque dans une ouverture d'une paroi ventriculaire d'un cœur battant, simple dans sa conception, permettant d'éviter l'obstruction des orifices d'aspiration du sang de la pompe cardiaque, de maintenir cette dernière dans la cavité ventriculaire selon une orientation souhaitée et de permettre le positionnement de l'orifice d'éjection de la pompe cardiaque à une distance contrôlée de la valve aortique.

Un autre objet de la présente invention est un revêtement dont les propriétés permettent de faciliter un recouvrement tissulaire et de rigidifier un ancrage du corps principal dudit dispositif de fixation.

Encore un objet de la présente invention est l'emploi d'une couronne lisse empêchant toute agrégation protéiforme, cellulaire ou moléculaire, empêchant la colonisation du corps de la pompe par un tissu cellulaire de recouvrement au sein du ventricule.

### Exposé de l'invention

Le présent document concerne un dispositif de fixation d'une pompe cardiaque dans une ouverture d'une paroi ventriculaire d'un cœur battant, comprenant
- un corps principal creux de forme générale cylindrique présentant une surface externe,
- ce corps principal creux comprenant une extrémité proximale et une extrémité distale entre lesquelles s'étend ladite surface externe, ladite extrémité distale étant destinée à former saillie de ladite paroi ventriculaire à l'intérieur de la cavité ventriculaire correspondante du cœur battant,
- au moins une portion de la surface externe dudit corps principal destinée à être placée à l'intérieur de ladite cavité ventriculaire lorsque l'extrémité proximale de ce dispositif de fixation est fixée sur ladite paroi ventriculaire, à l'exclusion de son extrémité distale, présente un relief de surface pourvue d'excroissances et de creux réalisé dans un matériau autorisant l'adhésion et la croissance de cellules endothéliales, au moins ladite portion de surface externe comprenant un revêtement recouvrant une surface de titane ou d'alliage de titane,
- l'extrémité distale dudit corps principal creux forme une couronne lisse ayant une rugosité moyenne arithmétique Rₘₐₓ inférieur ou égal à 1 µm pour stopper la colonisation dudit dispositif de fixation par des cellules endothéliales.

La couronne lisse permet ainsi de créer une barrière à la colonisation générant un espacement libre de tout tissu naturel. Il résulte de cela que les ouvertures de la pompe cardiaque ne sont plus obstruées et donc que la pompe cardiaque n'est plus bouchée.

Une endothélisation du revêtement du corps principal est contrôlée par l'état de surface dudit revêtement. Par endothélisation, on entend une colonisation par un tissu cellulaire naturel. Le revêtement du corps principal, grâce à un relief de surface pourvue d'excroissances et de creux, favorise cette endothélisation. Celle-ci est d'autant plus favorisée que le revêtement est en titane ou en un alliage de titane. En effet, par cette technique, l'adhésion cellulaire est améliorée.

Une telle endothélisation a pour avantage de garantir un ancrage et une bonne orientation d'un ensemble corps principal - pompe cardiaque. En effet, cette endothélisation permet d'exercer une pression sur l'ensemble corps principal - pompe cardiaque. Cette notion d'orientation du corps de la pompe cardiaque est très importante car cela permet non seulement de bien maintenir la pompe cardiaque en place, mais aussi de bien l'agencer et la maintenir stable en regard de la valve aortique. La pompe est ainsi bloquée à la profondeur désirée dans le ventricule.

Grâce à cette endothélisation, le revêtement du corps principal est en outre préservé des attaques bactériennes éventuelles. La quantité de tissu cellulaire s'agglomérant sur le revêtement est optimisée en fonction de la géométrie du cœur, selon que ce cœur ait un apex obtus ou oblique.

Ledit corps principal creux comporte un premier corps cylindrique creux réalisé entièrement en titane ou en alliage de titane, ledit premier corps cylindrique comportant sur au moins une partie de sa surface externe ledit revêtement de surface, un second corps cylindrique creux présentant une collerette externe à une extrémité, ledit second corps cylindrique étant inséré dans ledit premier corps cylindrique creux de sorte que son extrémité soit placée dans le prolongement de ladite surface externe du premier corps cylindrique en formant une continuité de surface avec celle-ci, ladite extrémité du second corps cylindrique définissant l'extrémité distale dudit corps principal.

Cet agencement permet non seulement de bien maintenir la pompe cardiaque en place, mais aussi de bien l'agencer et la maintenir stable en regard de la valve aortique.

Ladite extrémité distale ou collerette externe peut présenter une dimension longitudinale comprise entre 10 mm et 20 mm.

La quantité de tissu cellulaire s'agglomérant sur le revêtement est optimisée en fonction de la géométrie du cœur. Grâce à cette dimension longitudinale, le cas d'un cœur obtu est couvert.

L'extrémité distale ou collerette externe peut présenter une dimension longitudinale comprise entre 2 mm et 10 mm.

La quantité de tissu cellulaire s'agglomérant sur le revêtement est optimisée en fonction de la géométrie du cœur. Grâce à cette dimension longitudinale, le cas d'un cœur oblique est couvert.

Ledit second corps cylindrique est lisse et réalisée entièrement en PEEK (polyétheréthercétone).

Lorsque le second corps est réalisé en PEEK, il est avantageusement hydrophobe et inerte. Ce second corps est d'autant plus hydrophobe et inerte que sa surface est lisse. Ce second corps ne supporte pas d'adhésion cellulaire.

Une rugosité moyenne arithmétique du revêtement recouvrant la surface externe de titane ou d'un alliage de titane peut être comprise entre 100 µm et 300 µm.

Le revêtement du corps principal, grâce à l'augmentation de l'aire de contact ou plus précisément à la forte rugosité moyenne arithmétique, favorise l'endothélisation.

L'extrémité proximale dudit corps principal peut comporter une forme évasée délimitant un logement pour recevoir une bague de serrage, laquelle a pour fonction de pincer annulairement le corps de la pompe cardiaque, la paroi intérieure de ladite extrémité proximale présentant de plus un premier filetage intérieur pour visser un écrou dentelé.

Cette bague de serrage permet de réaliser une étanchéité et un maintien de l'écrou dentelé.

Une paroi extérieure de l'extrémité proximale peut comporter un second filetage extérieur pour recevoir une bague comprenant au moins une oreille, de préférence quatre (4), comportant chacune un orifice de réception de l'extrémité d'un outil de serrage.

De manière avantageuse, cette bague facilite le serrage de l'écrou dentelé pour l'opérateur. L'outil de serrage comprenant une empreinte destinée à coopérer avec l'écrou dentelé, tel que des dents complémentaires des dents de l'écrou dentelé, l'extrémité libre de cet outil de serrage peut être insérée dans l'ouverture d'une oreille de sorte que son empreinte soit en prise avec des dents de l'écrou dentelé pour le serrage de cet écrou.

Par ailleurs, l'extrémité distale dudit corps principal peut être chanfreinée pour assurer un pincement du corps de la pompe cardiaque insérée dans ledit dispositif lorsque ladite au moins une partie de la surface externe du corps principal a été colonisée par des cellules endothéliales. Le corps de la pompe est ainsi maintenu en position de manière ferme.

La géométrie de cette extrémité distale chanfreinée permet de s'adapter à la géométrie du cœur battant et de permettre une meilleure orientation de la pompe cardiaque.

Le revêtement peut être uniquement formé de microsphères de titane.

Ces microsphères de titane facilitent un recouvrement tissulaire et solidifient ou rigidifient l'ancrage du corps principal.

Les microsphères de titane peuvent présenter chacune un diamètre moyen compris entre 100 µm et 300 µm.

Cette distribution de taille de diamètre est calculée pour augmenter le plus possible la rugosité moyenne arithmétique du revêtement.

Le revêtement peut alternativement comporter un tissu ajouré formé d'une pluralité de filaments de polyesther.

Le revêtement peut être de type Spondycoat ^{®} - T317A.

La surface externe du revêtement peut encore présenter un état de surface décapé. On entend par « état de surface décapé », un état de surface pour lequel une couche de matière de la surface externe est éliminé, laissant apparent un substrat. Par exemple, l'état de cette surface pourrait être celui résultant d'un sablage de la surface externe du revêtement. Lors de ce sablage, un abrasif est projeté à grande vitesse à l'aide d'air comprimé au travers d'une buse, sur la surface externe à décaper.

Le revêtement peut comporter des excroissances et des creux avec une répartition aléatoire.

### Brève description des dessins

D'autres caractéristiques, détails et avantages apparaîtront à la lecture de la description détaillée ci-après, et à l'analyse des dessins annexés, sur lesquels :
**Fig. 1**
   [Fig. 1] montre une pompe cardiaque selon l'art antérieur.
**Fig. 2**
   [Fig. 2] montre une première vue d'un assemblage d'un dispositif de fixation selon l'invention.
**Fig. 3**
   [Fig. 3] montre une deuxième vue d'un assemblage partiellement monté du dispositif de fixation illustré en figure 2, selon l'invention.
**Fig. 4**

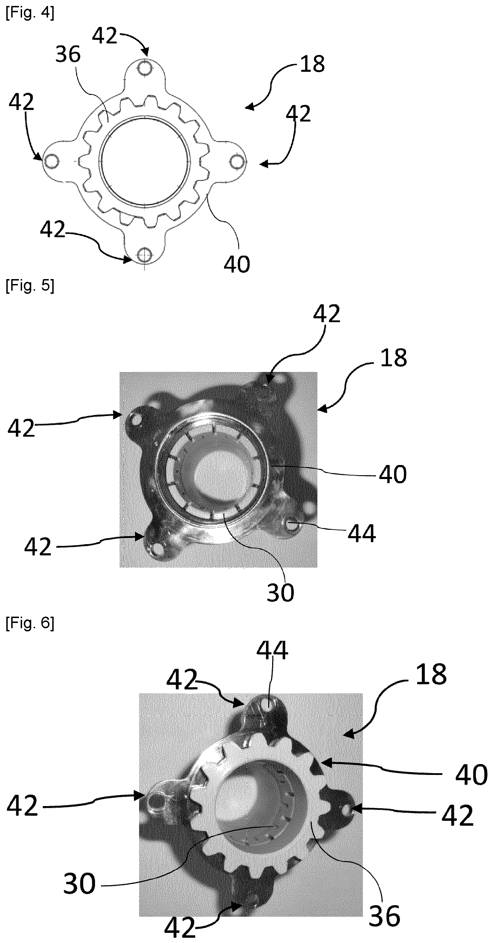

   [Fig. 4] montre une vue depuis une bague et un écrou dentelé du dispositif de fixation, selon l'invention.
**Fig. 5**
   [Fig. 5] montre une vue selon la figure 4 du dispositif de fixation montrant un positionnement d'une bague de serrage, selon l'invention.
**Fig. 6**
   [Fig. 6] montre une vue aval du dispositif de fixation avec l'écrou dentelé monté dans ledit dispositif de fixation, selon l'invention.
**Fig. 7**

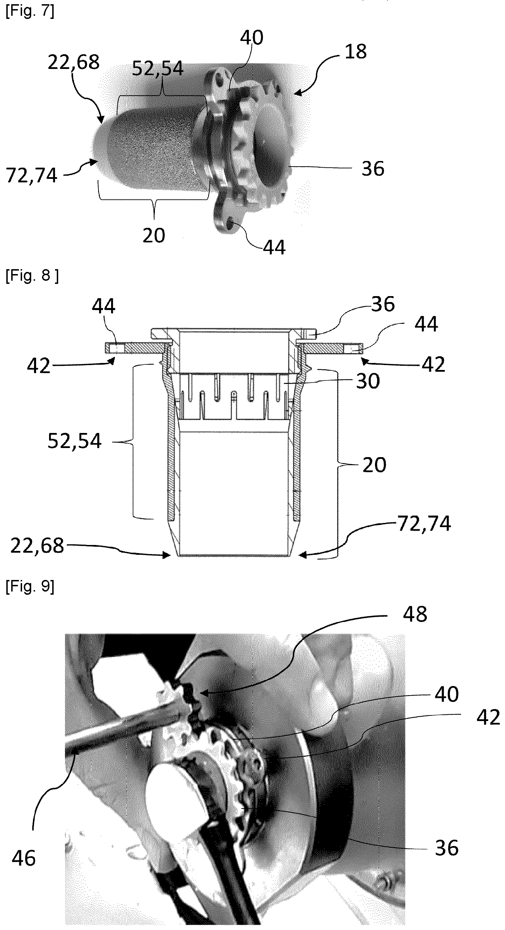

   [Fig. 7] montre une vue de côté du dispositif de fixation.
**Fig. 8**
   [Fig. 8] montre une vue en coupe et schématique du dispositif de fixation.
**Fig. 9**
   [Fig. 9] montre un montage de l'écrou dentelé contre la bague du dispositif de fixation.
**Fig. 10**

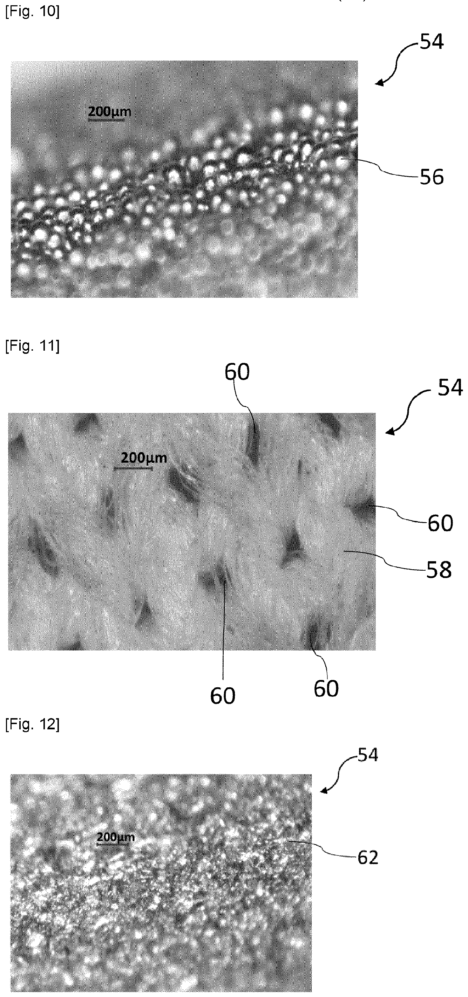

   [Fig. 10] montre un premier mode de réalisation d'un revêtement d'un tube de fixation, selon l'invention.
**Fig. 11**
   [Fig. 11] montre un deuxième mode de réalisation d'un revêtement d'un tube de fixation, selon l'invention.
**Fig. 12**
   [Fig. 12] montre un troisième mode de réalisation d'un revêtement d'un tube de fixation, selon l'invention.
**Fig. 13**

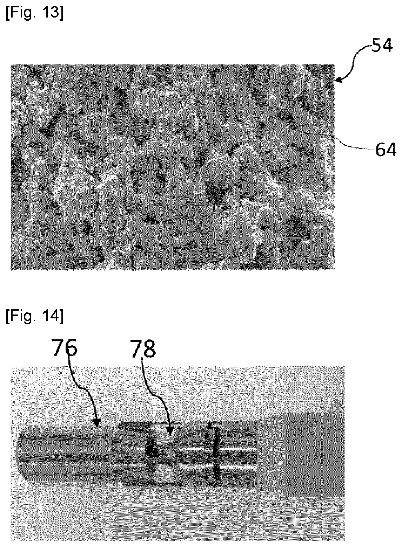

   [Fig. 13] montre un quatrième mode de réalisation d'un revêtement d'un tube de fixation, selon l'invention.
**Fig. 14**
   [Fig. 14] montre une pompe cardiaque selon l'invention.

### Description détaillée

Les dessins et la description ci-après contiennent, pour l'essentiel, des éléments de caractère certain. Ils pourront donc non seulement servir à mieux faire comprendre la présente invention, mais aussi contribuer à sa définition, le cas échéant. On note que les figures ne sont pas à l'échelle.

Le présent document concerne un dispositif de fixation 18 d'une pompe cardiaque dans une ouverture d'une paroi ventriculaire d'un cœur battant.

Comme illustré en figures 2 à 8, le dispositif de fixation 18 comporte un corps principal 20 creux de forme générale cylindrique. Ce corps principal 20 comprend une extrémité distale 22 et une extrémité proximale 24. Par extrémité distale 22, on entend l'extrémité du corps principal 20 creux la plus éloignée de la paroi ventriculaire du cœur battant. A l'inverse, par extrémité proximale 24, on entend l'extrémité du corps principal 20 creux la plus proche de la paroi ventriculaire du cœur battant. Le corps principal 20 creux comprend un premier corps cylindrique 26 creux et un second corps cylindrique 28 creux. Le premier corps cylindrique 26 creux est réalisé en titane ou en un alliage de titane. Le second corps cylindrique 28 creux est réalisé entièrement en céramique ou en PEEK (polyétheréthercétone).

Le premier corps cylindrique 26 du corps principal 20 comprend, à l'extrémité proximale 24, une forme évasée délimitant un logement intérieur. La forme évasée peut, par exemple, être de forme conique. Le logement délimite un espace à l'intérieur du premier corps cylindrique 26 du corps principal 20 apte à recevoir une bague de serrage 30. Cette bague de serrage 30 est apte à se déformer pour venir épouser une paroi intérieure du premier corps cylindrique 26 du corps principal 20 à proximité de ladite extrémité proximale 24.

Le premier corps cylindrique 26 comporte à l'extrémité proximale 24 un premier filetage intérieur 32 et un second filetage extérieur 34. Le premier filetage intérieur 32 du premier corps cylindrique 26 est configuré pour recevoir un écrou dentelé 36 apte à venir se visser dans ledit premier filetage intérieur 32 et à venir au contact d'une extrémité 38 de la bague de serrage 30. Le second filetage extérieur 34 du premier corps cylindrique 26 est configuré pour recevoir une bague 40. Cette bague 40 est apte à venir se visser le long dudit second filetage extérieur 34. La bague 40 comporte quatre oreilles 42. Ces oreilles 42 comportent chacune un orifice de réception 44 d'un outil de serrage 46. Comme illustré en figure 8, l'outil de serrage 46 comporte une extrémité libre 48 venant coopérer avec l'écrou dentelé 36.

Une surface externe 52 du premier corps cylindrique 26, excluant l'extrémité distale 22 de ce premier corps cylindrique 26, comporte un revêtement 54. Ce revêtement 54 présente un relief de surface pourvu d'une répartition aléatoire d'excroissances et de creux. Ce revêtement recouvrant la surface externe 52 du premier corps cylindrique 26 à l'exception de l'extrémité distale 22 présente un paramètre de rugosité moyenne arithmétique compris entre 100 µm et 300 µm.

Dans un mode de réalisation particulier illustré en figure 10, ce revêtement 54 peut comprendre une pluralité de couches de microsphères 56 de titane. Ces microsphères 56 de titane ont un diamètre moyen compris entre 100 µm et 300 µm. Ce revêtement 54 présente un relief de surface caractérisé par une rugosité moyenne arithmétique comprise entre 100 et 300 µm. Ces microsphères 56 sont projetées sur la surface externe 52 du premier corps cylindrique 26 excluant l'extrémité distale. Les microsphères 56 sont liées à la surface externe 52 par chauffage à une température proche de la température de fusion du titane. Aucun liant n'est employé pour maintenir les microsphères 56 entre elles.

Dans un mode de réalisation particulier illustré en figure 11, ce revêtement 54 peut comprendre un tissu 58 ajouré formé d'une pluralité de filament de polyester. Les filaments ont un diamètre moyen compris entre 250 µm et 350 µm. Ce tissu comporte des ouvertures 60 d'un diamètre moyen compris entre 50 µm et 100 µm.

Dans un mode de réalisation particulier illustré en figure 12, le revêtement 54 peut comporter un état de surface décapé 62. Il résulte de ce décapage qu'une granulosité est présente à la surface augmentant l'aire de contact entre ledit revêtement et un sang circulant dans le ventricule gauche du cœur. Cette granulosité peut être quantifier en termes de rugosité moyenne arithmétique. La rugosité moyenne arithmétique dudit revêtement est comprise entre 100 et 300 µm. Ce décapage est obtenu par sablage.

Dans un mode de réalisation particulier illustré en figure 13, la surface externe a reçu un traitement de surface. Le revêtement est constitué de PEEK ayant reçu une projection de plasma. Classiquement, le plasma est un gaz partiellement ionisé composé d'atomes, de molécules, d'ions et de radicaux libres excités, suite à une stimulation par radio fréquences, micro-ondes ou une décharge d'électrons. Cette projection de plasma est configuré pour influencer un caractère hydrophile/hydrophobe, une charge et une rugosité de surface. Le revêtement peut être de type Spondycoat ^{®} 64 - T317A.

Le second corps cylindrique 28 est apte à venir s'insérer à l'intérieur du premier corps cylindrique 26 creux de sorte qu'une première partie 66 soit au contact contre une surface interne du second corps cylindrique 26 creux et une seconde partie 68 soit en saillie d'une extrémité distale 66 du premier corps cylindrique 26 creux, cette extrémité distale 70 du premier corps cylindrique 26 étant du côté opposé au second filetage extérieur 34. Cette seconde partie 68 du second corps cylindrique 28 forme l'extrémité distale 22 du corps principal 20. Cette seconde partie 68 comporte une collerette externe 72. Cette collerette externe 72 comprend une couronne lisse 74. Cette couronne lisse 74 a une rugosité moyenne arithmétique Rₘₐₓ inférieur ou égal à 1 µm pour stopper la colonisation dudit dispositif de fixation 18 par des cellules endothéliales. L'extrémité distale 22 du corps principal 20 et donc la couronne lisse 74 est chanfreinée. Cette couronne lisse 74 chanfreinée est configurée pour pincer la pompe cardiaque 76. Comme illustré en figure 14, la pompe cardiaque 76 est insérée du côté de l'écrou dentelé 36, passe à l'intérieur du corps principal 20 et ressort du côté de la couronne lisse 74.

Une dimension longitudinale sur laquelle s'étend la couronne lisse 74 dépend d'une envergure du cœur et d'une épaisseur d'une paroi dudit coeur. Par dimension longitudinale, on entend un espacement entre l'extrémité distale 70 du premier corps cylindrique 26 et une extrémité distale 76 de la couronne lisse 74. On peut également parler d'une profondeur de la couronne lisse 74.

La profondeur de la couronne lisse est comprise entre 2 et 10 mm, si le cœur a un apex très obtus en fin de contraction. En effet, pendant la contraction, il y a alors très peu de contact entre la couronne lisse et la paroi du cœur.

En revanche, la profondeur de la couronne lisse 74 est comprise entre 10 et 20 mm, si le cœur a un apex très aigu en fin de contraction. En effet, l'augmentation de la profondeur de la couronne lisse 74 est configurée pour éviter que les parois du cœur ne soient en contact avec cette couronne lisse 74. Il n'y a ainsi aucun dépôt de cellules sur ladite couronne lisse 74.

En fonctionnement, la couronne lisse 74 permet de créer une barrière à la colonisation générant un espacement libre de tout tissu naturel. En effet, la couronne lisse 74 en PEEK est hydrophobe et inerte. Cette couronne lisse 74 est d'autant plus hydrophobe et inerte que sa surface est polie, l'extrémité distale la couronne lisse 74 ne supportera pas d'adhésion cellulaire. Il résulte de cela que des ouvertures 78 de la pompe cardiaque 76 ne sont plus obstruées et donc que la pompe cardiaque 76 n'est plus bouchée.

Une endothélisation du revêtement 54 du premier corps cylindrique 26 est contrôlé par l'état de surface dudit revêtement 54. Par endothélisation, on entend une colonisation par un tissu cellulaire naturel. Le revêtement 54 du premier corps cylindrique 26, grâce à l'augmentation de l'aire de contact ou plus précisément à la forte rugosité moyenne arithmétique, favorise l'endothélisation. En effet, par cette technique, l'adhésion cellulaire est améliorée. Cette endothélisation a pour avantage de renforcer la bonne orientation d'un système corps principal 20 - pompe cardiaque 76. En effet, cette endothélisation permet d'exercer une pression sur l'ensemble corps principal 20 - pompe cardiaque 76. Cette notion d'orientation du corps de la pompe cardiaque 76 est très importante car cela permet non seulement de bien maintenir la pompe cardiaque 76 en place, mais aussi de bien l'agencer et la maintenir stable en regard d'une valve aortique. Grâce à cette endothélisation, le revêtement 54 du premier corps cylindrique 26 est préservé des attaques bactériennes éventuelles. La quantité de tissu cellulaire s'agglomérant sur le revêtement 54 est optimisée en fonction de la géométrie du cœur, selon que ce cœur ait un apex obtus ou oblique.

L'emploi du second corps cylindrique 28 permet d'éviter de rayer la pompe cardiaque 76 en employant un matériau plus tendre.

## Revendications

1. Dispositif de fixation (18) d'une pompe cardiaque (76) dans une ouverture d'une paroi ventriculaire d'un cœur battant, comprenant
- un corps principal (20) creux de forme générale cylindrique présentant une surface externe (52),
- ce corps principal (20) creux comprenant une extrémité proximale (24) et une extrémité distale (22) entre lesquelles s'étend ladite surface externe (52), ladite extrémité distale (22) étant destinée à former saillie de ladite paroi ventriculaire à l'intérieur de la cavité ventriculaire correspondante du cœur battant,
- au moins une portion de la surface externe (52) dudit corps principal (20) destinée à être placée à l'intérieur de ladite cavité ventriculaire, à l'exclusion de son extrémité distale (22), présente un relief de surface pourvue d'excroissances et de creux réalisé dans un matériau autorisant l'adhésion et la croissance de cellules endothéliales, au moins ladite portion de surface externe (52) comprenant un revêtement (54) recouvrant une surface de titane ou d'alliage de titane,
- l'extrémité distale (22) dudit corps principal creux forme une couronne lisse (74) ayant une rugosité moyenne arithmétique Rₘₐₓ inférieur ou égal à 1 µm pour stopper la colonisation dudit dispositif de fixation par des cellules endothéliales,
- ledit corps principal (20) creux comporte un premier corps cylindrique (26) creux réalisé entièrement en titane ou en alliage de titane, ledit premier corps cylindrique (26) comportant sur au moins une partie de sa surface externe (52) ledit revêtement (54) de surface, un second corps cylindrique (28) creux présentant une collerette externe (72) à son extrémité, ledit second corps cylindrique (28) étant inséré dans ledit premier corps cylindrique (26) creux de sorte que son extrémité soit placée dans le prolongement de ladite surface externe (52) du premier corps cylindrique (26) en formant une continuité de surface avec celle-ci, ladite extrémité du second corps cylindrique (28) définissant l'extrémité distale (22) dudit corps principal (20), le dispositif de fixation étant **caractérisé en ce que** le -ee-second corps est réalisé en PEEK et présente une surface lisse ayant une rugosité moyenne arithmétique Rmax inférieur ou égal à 1 µm.

2. Dispositif selon la revendication 1, dans lequel ladite extrémité distale (22) ou collerette externe (72) présente une dimension longitudinale comprise entre 10 mm et 20 mm.

3. Dispositif selon la revendication 1, dans lequel l'extrémité distale (22) ou collerette externe (72) présente une dimension longitudinale comprise entre 2 mm et 10 mm.

4. Dispositif selon l'une des revendications précédentes, dans lequel une rugosité moyenne arithmétique du revêtement (54) recouvrant la surface externe (52) de titane ou d'un alliage de titane est comprise entre 100 µm et 300 µm.

5. Dispositif selon l'une des revendications précédentes, dans lequel l'extrémité proximale (24) dudit corps principal (20) comporte une forme évasée délimitant un logement pour recevoir une bague de serrage (30) pour pincer annulairement le corps de la pompe cardiaque (76), la paroi intérieure de ladite extrémité proximale (24) présentant un premier filetage intérieur (32) pour visser un écrou dentelé (36).

6. Dispositif selon l'une des revendications précédentes, dans lequel une paroi extérieure de l'extrémité proximale (24) comporte un second filetage extérieur (34) pour recevoir une bague (40) comprenant au moins une oreille (42) comportant un orifice (44) de réception de l'extrémité d'un outil de serrage (46).

7. Dispositif selon l'une des revendications précédentes, dans lequel l'extrémité distale (22) dudit corps principal est chanfreinée pour pincer le corps de la pompe cardiaque (76) insérée dans ledit dispositif lorsque ladite au moins une partie de la surface externe (52) du corps principal (20) a été colonisée par des cellules endothéliales.

8. Dispositif selon l'une des revendications précédentes, dans lequel le revêtement (54) est uniquement formé de microsphères (56) de titane.

9. Dispositif selon la revendication 8, dans lequel les microsphères (56) de titane présentent chacune un diamètre moyen compris entre 100 µm et 300 µm.

10. Dispositif selon l'une des revendications 1 à 7, dans lequel le revêtement (54) comporte un tissu (58) ajouré formé d'une pluralité de filaments de polyester.

11. Dispositif selon l'une des revendications 1 à 7, dans lequel le revêtement (54) est de type Spondycoat ^{®} (64) - T317A.

12. Dispositif selon l'une des revendications 1 à 7, dans lequel la surface externe du revêtement (54) comporte un état de surface décapé (62).

13. Dispositif selon l'une des revendications précédentes, dans lequel le revêtement (54) comporte des excroissances et des creux avec une répartition aléatoire.

## Patentansprüche

1. Vorrichtung zur Befestigung (18) einer Herzpumpe (76) in einer Öffnung einer Ventrikelwand eines schlagenden Herzens, umfassend
- einen hohlen Hauptkörper (20) von allgemein zylindrischer Form, der eine Außenfläche (52) aufweist,
- wobei dieser hohle Hauptkörper (20) ein proximales Ende (24) und ein distales Ende (22) umfasst, zwischen denen sich die Außenfläche (52) erstreckt, wobei das distale Ende (22) dazu bestimmt ist, aus der Ventrikelwand in den entsprechenden Ventrikelraum des schlagenden Herzens vorzustehen,
- wobei wenigstens ein Teil der Außenfläche (52) des Hauptkörpers (20), der im Inneren des Ventrikelraums platziert werden soll, mit Ausnahme seines distalen Endes (22) ein Relief einer mit Ausstülpungen und Vertiefungen versehenen Oberfläche aufweist, das aus einem Material besteht, das die Adhäsion und das Wachstum von Endothelzellen ermöglicht, wobei wenigstens der Abschnitt der Außenfläche (52) eine Beschichtung (54) umfasst, die eine Oberfläche aus Titan oder einer Titanlegierung bedeckt,
- wobei das distale Ende (22) des hohlen Hauptkörpers eine glatte Krone (74) bildet, mit einer mittleren arithmetischen Rauheit Rₘₐₓ von kleiner gleich 1 µm, um die Besiedlung der Befestigungsvorrichtung durch Endothelzellen zu verhindern,
- wobei der hohle Hauptkörper (20) einen ersten hohlen zylindrischen Körper (26) umfasst, der vollständig aus Titan oder einer Titanlegierung gefertigt ist, wobei der erste zylindrische Körper (26) an wenigstens einem Teil seiner Außenfläche (52) die Oberflächenbeschichtung (54) aufweist, wobei ein zweiter hohler zylindrischer Körper (28) an seinem Ende einen Außenkragen (72) aufweist, wobei der zweite zylindrische Körper (28) in den ersten hohlen zylindrischen Körper (26) derart eingesetzt ist, dass sein Ende in der Verlängerung der Außenfläche (52) des ersten zylindrischen Körpers (26) angeordnet ist und mit dieser eine Oberflächenkontinuität bildet, wobei das Ende des zweiten zylindrischen Körpers (28) das distale Ende (22) des Hauptkörpers (20) definiert,
wobei die Befestigungsvorrichtung **dadurch gekennzeichnet ist, dass** der zweite Körper aus PEEK besteht und eine glatte Oberfläche mit einer mittleren arithmetischen Rauheit Rmax von kleiner gleich 1 µm aufweist.

2. Vorrichtung nach Anspruch 1, wobei das distale Ende (22) oder der Außenkragen (72) eine Längsabmessung zwischen 10 mm und 20 mm aufweist.

3. Vorrichtung nach Anspruch 1, wobei das distale Ende (22) oder der Außenkragen (72) eine Längsabmessung zwischen 2 mm und 10 mm aufweist.

4. Vorrichtung nach einem der vorhergehenden Ansprüche, wobei eine mittlere arithmetische Rauheit der Beschichtung (54), die die Außenfläche (52) mit Titan oder einer Titanlegierung bedeckt, zwischen 100 µm und 300 µm liegt.

5. Vorrichtung nach einem der vorhergehenden Ansprüche, wobei das proximale Ende (24) des Hauptkörpers (20) eine konisch erweiterte Form aufweist, die eine Aussparung zur Aufnahme eines Klemmrings (30) zum ringförmigen Einklemmen des Herzpumpenkörpers (76) begrenzt, wobei die Innenwand des proximalen Endes (24) ein erstes Innengewinde (32) zum Aufschrauben einer Zahnmutter (36) aufweist.

6. Vorrichtung nach einem der vorhergehenden Ansprüche, wobei eine Außenwand des proximalen Endes (24) ein zweites Außengewinde (34) zur Aufnahme eines Rings (40) aufweist, der wenigstens eine Öse (42) mit einer Öffnung (44) zur Aufnahme des Endes eines Spannwerkzeugs (46) umfasst.

7. Vorrichtung nach einem der vorhergehenden Ansprüche, wobei das distale Ende (22) des Hauptkörpers abgeschrägt ist, um den Körper der in die Vorrichtung eingesetzten Herzpumpe (76) einzuklemmen, wenn wenigstens ein Teil der Außenfläche (52) des Hauptkörpers (20) von Endothelzellen besiedelt wurde.

8. Vorrichtung nach einem der vorhergehenden Ansprüche, wobei die Beschichtung (54) nur aus Mikrokugeln aus Titan (56) besteht.

9. Vorrichtung nach Anspruch 8, wobei die Mikrokugeln aus Titan (56) jeweils einen mittleren Durchmesser zwischen 100 µm und 300 µm aufweisen.

10. Vorrichtung nach einem der Ansprüche 1 bis 7, wobei die Beschichtung (54) ein durchbrochenes Gewebe (58) umfasst, das aus einer Vielzahl von Polyesterfilamenten gebildet ist.

11. Vorrichtung nach einem der Ansprüche 1 bis 7, wobei die Beschichtung (54) vom Typ Spondycoat^{®} (64)-T317A ist.

12. Vorrichtung nach einem der Ansprüche 1 bis 7, wobei die Außenfläche der Beschichtung (54) eine gebeizte Oberflächenbeschaffenheit (62) aufweist.

13. Vorrichtung nach einem der vorhergehenden Ansprüche, wobei die Beschichtung (54) zufällig verteilte Erhebungen und Vertiefungen aufweist.

## Claims

1. A device (18) for attaching a heart pump (76) in an opening of a ventricular wall of a beating heart, comprising
- a hollow main body (20) of general cylindrical shape having an outer surface (52),
- this hollow main body (20) comprising a proximal end (24) and a distal end (22) between which said outer surface (52) extends, said distal end (22) being intended to form a protrusion of said ventricular wall inside the corresponding ventricular cavity of the beating heart,
- at least one portion of the outer surface (52) of said main body (20) intended to be placed inside said ventricular cavity, excluding the distal end (22) thereof, has a surface relief provided with outgrowths and hollows made of a material enabling the adhesion and growth of endothelial cells, at least said outer surface portion (52) comprising a coating (54) covering a titanium or titanium alloy surface,
- the distal end (22) of said hollow main body forms a smooth crown (74) having an arithmetic mean roughness Rₘₐₓ which is less than or equal to 1 µm to stop the colonisation of said attachment device by endothelial cells,
- said hollow main body (20) includes a first hollow cylindrical body (26) made entirely of titanium or titanium alloy, said first cylindrical body (26) including said surface coating (54) on at least one portion of the outer surface (52) thereof, a second hollow cylindrical body (28) having an outer flange (72) at the end thereof, said second cylindrical body (28) being inserted into said first hollow cylindrical body (26) such that the end thereof is placed in the extension of said outer surface (52) of the first cylindrical body (26) forming a surface continuity therewith, said end of the second cylindrical body (28) defining the distal end (22) of said main body (20), the attachment device being **characterised in that** the second body is made of PEEK and has a smooth surface having an arithmetic mean roughness Rmax which is less than or equal to 1 µm.

2. The device according to claim 1, wherein said distal end (22) or outer flange (72) has a longitudinal dimension comprised between 10 mm and 20 mm.

3. The device according to claim 1, wherein the distal end (22) or outer flange (72) has a longitudinal dimension comprised between 2 mm and 10 mm.

4. The device according to one of the preceding claims, wherein an arithmetic mean roughness of the coating (54) covering the outer surface (52) of titanium or of a titanium alloy is comprised between 100 µm and 300 µm.

5. The device according to one of the preceding claims, wherein the proximal end (24) of said main body (20) includes a flared shape delimiting a housing to receive a clamping ring (30) for annularly pinching the body of the heart pump (76), the inner wall of said proximal end (24) having a first inner thread (32) for screwing a serrated nut (36).

6. The device according to one of the preceding claims, wherein an outer wall of the proximal end (24) includes a second outer thread (34) for receiving a ring (40) comprising at least one ear (42) including an orifice (44) for receiving the end of a clamping tool (46).

7. The device according to one of the preceding claims, wherein the distal end (22) of said main body is chamfered to pinch the heart pump body (76) inserted into said device when said at least one portion of the outer surface (52) of the main body (20) has been colonised by endothelial cells.

8. The device according to one of the preceding claims, wherein the coating (54) is only formed of titanium microspheres (56).

9. The device according to claim 8, wherein the titanium microspheres (56) each have an average diameter comprised between 100 µm and 300 µm.

10. The device according to one of claims 1 to 7, wherein the coating (54) includes an openwork fabric (58) formed of a plurality of polyester filaments.

11. The device according to one of claims 1 to 7, wherein the coating (54) is of the Spondycoat^{®} (64) - T317A type.

12. The device according to one of claims 1 to 7, wherein the outer surface of the coating (54) includes a pickled surface condition (62).

13. The device according to one of the preceding claims, wherein the coating (54) includes outgrowths and hollows with a random distribution.
